Europäisches Patentamt

European Patent Office

Office européen des brevets

(11) Veröffentlichungsnummer: **0 035 709**
**B1**

(12) **EUROPÄISCHE PATENTSCHRIFT**

(45) Veröffentlichungstag der Patentschrift: **28.12.83**

(21) Anmeldenummer: **81101412.5**

(22) Anmeldetag: **26.02.81**

(51) Int. Cl.³: **B 01 J 8/02, C 07 C 29/15, C 07 C 31/04**

(54) **Methanol-Reaktor.**

(30) Priorität: **26.02.80 DE 3007202**

(43) Veröffentlichungstag der Anmeldung:
**16.09.81 Patentblatt 81/37**

(45) Bekanntmachung des Hinweises auf die Patenterteilung:
**28.12.83 Patentblatt 83/52**

(84) Benannte Vertragsstaaten:
**BE DE FR GB IT NL**

(56) Entgegenhaltungen:
**DE - A - 2 022 388**
**DE - A - 2 504 343**
**US - A - 2 248 993**

(73) Patentinhaber: **Linde Aktiengesellschaft**
**Abraham-Lincoln-Strasse 21**
**D-6200 Wiesbaden (DE)**

(72) Erfinder: **Lahne, Ulrich**
**Oskar-Coester-Weg 9**
**D-8000 München 71 (DE)**
Erfinder: **Hesse, Peter, Dr.**
**Helmpertstrasse 21**
**D-8000 München 21 (DE)**
Erfinder: **Kliem, Erhard**
**Margeritenstrasse 32**
**D-8190 Wolfratshausen (DE)**
Erfinder: **Kruis, Bernhard, Dr.**
**Wolfratshauser Strasse 98**
**D-8023 Pullach (DE)**
Erfinder: **Lohmüller, Reiner, Dr.**
**Bergstrasse 11a**
**D-8000 München 90 (DE)**

(74) Vertreter: **Schaefer, Gerhard, Dr.**
**Linde Aktiengesellschaft Zentrale Patentabteilung**
**D-8023 Höllriegelskreuth (DE)**

Courier Press, Leamington Spa, England.

## Methanol-Reaktor

Die Erfindung betrifft einen Reaktor zur Durchführung einer Methanolsynthese mit einem Gehäuse, einer in dem Gehäuse befindlichen Schüttung aus Katalysatorteilchen, sowie mit in der Katalysatorschüttung angeordneten, im wesentlichen quer zur Strömungsrichtung des Synthesegases verlaufenden Rohren zur Führung eines Kühlfluids.

Ein derartiger Reaktor ist in der DE—A—25 04 343 beschrieben worden. Er dient zur Durchführung exothermer katalytischer Reaktionen, bei denen aus einem Synthesegas unter Freiwerden von Wärme neue Reaktionsprodukte entstehen. Die Reaktionswärme wird bei diesem Reaktor durch ein Kühlfluid abgeführt, das in um ein Kernrohr gewickelten Rohren geführt ist.

Es hat sich gezeigt, daß der vorbekannte Reaktor insbesondere bei der stark exothermen Methanolsynthese nicht allen Anforderungen hinsichtlich des Wärmeübergangs vom Katalysator bzw. von den heißen Reaktionsprodukten auf die Kühlrohre gerecht werden konnte. Um die erforderliche Kühlfläche bereitstellen zu können, mußte die Anzahl und/oder die Oberfläche der Kühlrohre vergrößert werden, was zu sehr großen geometrischen Abmessungen des Reaktors sowie zu großem Materialbedarf und Gewicht führte.

Der vorliegenden Erfindung lag daher die Aufgabe zugrunde, einen Reaktor der eingangs genannten Art zu entwickeln, der besonders gut an die bei der Methanolsynthese herrschenden Bedingungen angepaßt ist und bei dem ein guter Wärmeübergang zwischen Katalysator und Reaktionsprodukten einerseits und dem Kühlfluid andererseits gewährleistet ist.

Diese Aufgabe wird erfindungsgemäß dadurch gelöst, daß der lichte Durchmesser der Rohre zwischen 4 und 50 mm beträgt, der Abstand a zweier benachbarter Rohre quer zur Strömungsrichtung des Synthesegases zwischen dem zweifachen und dem zwanzigfachen der kleinsten Längenabmessung eines Katalysatorteilchens, und der Abstand c zweier benachbarter Rohre in Strömungsrichtung des Synthesegases kleiner als 10a ist.

Die quer angeströmten Rohre des erfindungsgemäßen Methanolreaktors können beispielsweise in mehreren Lagen in radialem Abstand voneinander um ein Kernrohr gewickelt sein. Bei dieser Ausführungsform entspricht der Abstand a dem radialen lichten Abstand der Rohre und der Abstand $c_m$ dem axialen lichten Abstand der Rohre benachbarter Rohrwindungen. Es ist aber auch eine Anordnung von mehreren Reihen von Kühlrohren mit geradlinigen Abschnitten denkbar, bei der die Achsen der geradlinigen Rohrabschnitte im wesentlichen senkrecht zur Strömungsrichtung des Synthesegases liegen.

Als Längenabmessung eines Katalysatorteilchens sind beispielsweise die Kantenlänge bei quaderförmigen, die Höhe oder der Durchmesser bei zylinderförmigen und der Durchmesser bei kugelförmigen Katalysatorteilchen anzusehen.

Rohrdurchmesser und die Rohrabstände a und c werden innerhalb der angegebenen Bereiche so aufeinander abgestimmt, daß je nach den Reaktionsparametern—wie z.B. Temperatur des Kühlfluids, Zusammensetzung, Druck und Temperatur des Synthesegases, Art des Katalysators—eine optimale Anpassung des Methanolreaktors an den Reaktionsverlauf erreicht wird.

Mit dem erfindungsgemäßen Methanolreaktor läßt sich ein besonders guter Wärmeübergang von der Katalysatorschüttung und dem Reaktionsgas auf das Kühlfluid erreichen. Aus diesem Grund kommt der erfindungsgemäße Methanolreaktor mit erheblich weniger Kühlfläche als bisher bekannte Reaktoren aus und kann daher kleiner gebaut werden. Außerdem werden Materialkosten und Gewicht eingespart. Bei einer bevorzugten Ausführungsform des Erfindungsgegenstandes beträgt der Durchmesser der Rohre zwischen 10 und 30 mm.

Es hat sich als zweckmäßig erwiesen, wenn der Abstand a zwischen 10 und 40 mm und der Abstand c kleiner als 400 mmm ist.

Insbesondere ist es günstig, wenn der Abstand a zwischen 15 und 20 mm und der Abstand c kleiner als höchstens gleich 30 mm ist.

Eine besonders gute Anpassung an den Temperaturverlauf bei der Methanolsynthese läßt sich mit einer vorteilhaften Weiterbildung des Erfindungsgegenstandes erreichen, bei der sich der Abstand c der Rohre entlang der Strömungsrichtung des Synthesegases verändert.

Mit einer derartigen Konstruktion läßt sich auf einfache Weise die Kühlflächendichte im Reaktor verändern und genau dem örtlichen Wärmeanfall anpassen.

Die Variation des Abstandes c erfolgt bei einer zweckmäßigen Ausgestaltung des erfindungsgemäßen Reaktors stetig entlang des Strömungswegs des Synthesegases. Die stetige Änderung des Abstandes c kann sowohl eine gleichmäßige Zunahme oder Abnahme bedeuten, es kann aber auch sein, daß der Abstand c über die Länge des Reaktors ein oder mehrere Maximalwerte oder Minimalwerte annimmt.

Bei einer anderen bevorzugten Ausführungsform des Erfindungsgegenstandes ändert sich der Abstand c abschnittweise entlang des Strömungswegs des Synthesegases und ist innerhalb der Abschnitte konstant.

Weitere Ausgestaltungen des Erfindungsgegenstandes werden anhand schematisch dargestellter Ausführungsbeispiele näher erläutert.

Hierbei zeigen:

Figur 1 einen erfindungsgemäßen Methanolreaktor

Figur 2 einen Ausschnitt A aus Figur 1, die

Figuren 3, 4, 5 Ausschnitte B aus Figur 1, die jeweils verschiedene Modifikationen der Anordnung der Kühlrohre darstellt.

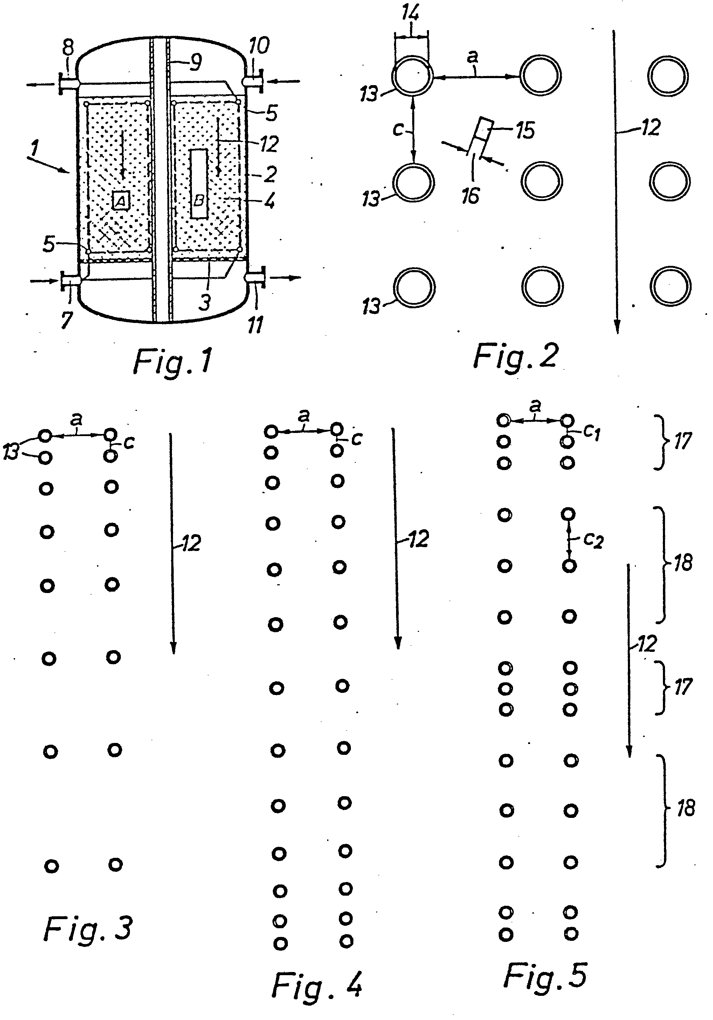

Der Methanolreaktor 1 gemäß Figur 1 weist ein äußeres Gehäuse 2 auf, in dem auf einer Lochplatte 3 eine Schüttung aus Katalysatorteilchen 4 ruht. In der Schüttung verlaufen Rohre 5 zur Führung eines Kühlfluids. Die Rohre 5 sind um ein Kernrohr 9 gewickelt. Die Enden der Rohre 5 münden in Stutzen 7, 8 zur Zu- und Abführung eines Kühlfluids. Im Gehäuse 2 sind außerdem Anschlußstutzen 10, 11 zur Zuführung des Synthesegases und zur Abführung des Methanols vorgesehen.

Das Synthesegas wird über Anschlußstutzen 10 in den Methanolreaktor geleitet. Es verteilt sich gleichmäßig über den Querschnitt des Reaktors und strömt in Richtung der Pfeile 12 durch die Katalysatorschüttung nach unten. Hierbei findet die Umsetzung zu Methanol statt. Die Reaktion ist stark exotherm. Um die Reaktionswärme abführen zu können, wird durch die in der Katalysatorschüttung 4 verlaufenden Rohre 5 ein Kühlfluid geleitet, das die Reaktionswärme aufnimmt und aus dem Methanolreaktor 1 ableitet.

Das Synthesegas strömt im wesentlichen quer zur Strömungsrichtung des Kühlfluids. Eine Queranströmung läßt sich auch mit einer anderen Rohranordnung erreichen, bei der ein Bündel von geradlinigen Rohren mit ihren Achsen senkrecht zur Strömungsrichtung des Synthesegases angeordnet sind. Für eine solche Rohranordnung gelten die nachstehenden Ausführungen in analoger Weise.

Figur 2 zeigt einen Ausschnitt A aus Figur 1. Die mit 13 bezeichneten Rohre zur Führung des Kühlfluids können jedoch prinzipiell auch zu einem Bündel geradliniger Rohre gehören. Entscheidend ist, daß die Rohre 13 von dem Synthesegas im wesentlich quer angeströmt werden (Pfeilrichtung 12). Die Rohre 13 sind von einer Schüttung aus Katalysatorteilchen umgeben, von denen stellvertretend ein Teilchen 15 figürlich dargestellt ist. Die Katalysatorteilchen können beliebige Form haben, z.B. als Quader, Zylinder, Kugeln oder Raschigringe ausgebildet sein.

Erfindungsgemäß beträgt der lichte Durchmesser 14 der Rohre 13 zwischen 4 und 50 mm, vorzugsweise zwischen 10 und 30 mm. Der Abstand c zweier benachbarter Rohre 13 in Strömungsrichtung des Synthesegases ist kleiner als 10a, wobei a der Abstand zweier benachbarter Rohre quer zur Strömungsrichtung des Synthesegases ist. Der Abstand a liegt wiederum zwischen dem zweifachen und dem zwanzigfachen der kleinsten Längenabmessung 16 des Katalysatorteilchens 15. Der Abstand a beträgt zwischen 10 und 40 mm, vorzugsweise zwischen 15 und 20 mm. Der Abstand c ist kleiner als 400 mm, vorzugsweise kleiner oder höchstens gleich 30 mm.

Es wurde gefunden, daß der Wärmeübergang vom Katalysator und vom Reaktionsgas auf das Kühlfluid besonders groß ist, wenn der Rohrdurchmesser 14 und die Abstände a und c aus den angegebenen Bereichen gewählt werden.

Die Figuren 3, 4, 5 zeigen Ausschnitte B aus Figur 1 für verschiedene Anordnungen der Kühlrohre 13. Auch hier können die Rohre 13 Teil eines Bündels geradliniger Rohre sein, das vom Synthesegas in Querrichtung angeströmt wird (Teil 12).

Gemäß Figur 3 nimmt der Abstand c entlang der Strömungsrichtung des Synthesegases stetig zu, gemäß Figur 4 nimmt der Abstand c zunächst stetig zu und dann stetig wieder ab, und gemäß Figur 5 weisen die Rohre in Strömungsrichtung des Synthesegases in zweierlei Abschnitten 17 und 18 zwei verschiedene Abstände $c_1$ und $c_2$ auf, wobei in jedem Abschnitt 17, 18 die Rohrabstände $c_1$ bzw. $c_2$ konstant sind und sich beim Übergang von einem Abschnitt zum anderen sprungartig ändern. In allen drei dargestellten Beispielen bleibt der Abstand a entlang der Strömungsrichtung des Synthesegases konstant.

**Patentansprüche**

1. Reaktor zur Durchführung einer Methanolsynthese mit einem Gehäuse, einer in dem Gehäuse befindlichen Schüttung aus Katalysatorteilchen, sowie mit in der Katalysatorschüttung angeordneten, im wesentlichen quer zur Strömungsrichtung des Synthesegases verlaufenden Rohren zur Führung eines Kühlfluids, dadurch gekennzeichnet, daß der lichte Durchmesser der Rohre (5, 13) zwischen 4 und 50 mm beträgt, der Abstand a zweier benachbarter Rohre (5, 13) quer zur Strömungsrichtung des Synthesegases zwischen dem zweifachen und dem zwanzigfachen der kleinsten Längenabmessung eines Katalysatorteilchens (15), und der Abstand c zweier benachbarter Rohre (5, 13) in Strömungsrichtung des Synthesegases kleiner als 10a ist.

2. Reaktor nach Anspruch 1, dadurch gekennzeichnet, daß der lichte Durchmesser der Rohre (5, 13) zwischen 10 und 30 mm liegt.

3. Reaktor nach Anspruch 1 oder 2, dadurch gekennzeichnet, daß der Abstand a zwischen 10 und 40 mm, und der Abstand c kleiner als 400 mm.

4. Reaktor nach einem der Ansprüche 1 bis 3, dadurch gekennzeichnet, daß der Abstand a zwischen 15 und 20 mm und der Abstand c kleiner oder höchstens gleich 30 mm ist.

5. Reaktor nach einem der Ansprüche 1 bis 4, dadurch gekennzeichnet, daß sich der Abstand c der Rohre (5, 13) entlang der Strömungsrichtung des Synthesegases verändert.

6. Reaktor nach Anspruch 5, dadurch gekennzeichnet, daß die Variation des Abstandes c stetig entlang des Strömungswegs des Synthesegases erfolgt.

7. Reaktor nach Anspruch 5, dadurch gekennzeichnet, daß sich der Abstand c abschnittweise entlang des Strömungswegs des Synthesegases ändert und innerhalb der Abschnitte (17, 18) konstant ist.

## Revendications

1. Réacteur pour produire du méthanol par synthèse, comportant une enceinte une garniture formée de particules de catalyseur et disposée dans ladite enceinte, ainsi que des tubes disposés dans la garniture de catalyseur, orientés sensiblement transversalement par rapport à la direction du gaz de synthèse et appelés à canaliser un fluide de refroidissement, caractérisé en ce que le diamètre intérieur des tubes (5, 13) est compris entre 4 et 50 mm, que la distance (a) entre deux tubes (5, 13) voisins transversalement à la direction d'écoulement du gaz de synthèse est comprise entre deux fois et vingt fois la plus petite dimension longitudinale d'une particule de catalyseur (15), et que la distance (c) entre deux tubes (5, 13) voisins dans la direction de l'écoulement du gaz de synthèse est inférieure à 10a.

2. Reacteur selon la revendication 1, caractérisé en ce que le diamètre intérieur des tubes (5, 13) est compris entre 10 et 30 mm.

3. Réacteur selon la revendication 1 ou 2, caractérisé en ce que la distance (a) est comprise entre 10 et 40 mm, copendant que la distance (c) est inférieure à 400 mm.

4. Réacteur selon une des revendications 1 à 3, caractérisé en ce que la distance (a) est comprise entre 15 et 20 mm, copendant que la distance (c) est inférieure ou au plus égale à 30 mm.

5. Réacteur selon une des revendications 1 à 4, caractérisé en ce que la distance (c) entre les tubes (5, 13) varie dans la direction d'écoulement du gaz de synthèse.

6. Réacteur selon la revendication 5, caractérisé en ce que la variation de la distance (c) le long du trajet d'écoulement du gaz de synthèse est continue.

7. Réacteur selon la revendication 5, caractérisé en ce que la distance (c) varie le long du trajet d'écoulement du gaz de synthèse et est constante à l'intérieur des tronçons (17, 18).

## Claims

1. A reactor for carrying out a methanol synthesis, having a housing, a charge of catalyst particles located in the housing, and pipes for conveying a cooling fluid arranged in the catalyst charge and running essentially transversely to the direction of flow of the synthesis gas, characterised in that the inner diameter of the pipes (5, 13) is between 4 and 50 mm; that the distance a between two adjacent pipes (5, 13) transversely to the direction of flow of the synthesis gas, is between twice and twenty times the smallest longitudinal dimension of a catalyst particle (15); and that in the direction of flow of the synthesis gas, the spacing c between two adjacent pipes (5, 13) is less than 10a.

2. A reactor according to Claim 1, characterised in that the inner diameter of the pipes (5, 13) is between 10 and 30 mm.

3. A reactor according to Claim 1 or Claim 2, characterised in that the spacing a is between 10 and 40 mm and the spacing c is less than 400 mm.

4. A reactor according to one of Claims 1 to 3, characterised in that the spacing a is between 15 and 20 mm and the spacing c is less than, or at most equals, 30 mm.

5. A reactor according to one of Claims 1 to 4, characterised in that the spacing c of the pipes (5, 13) varies along the direction of flow of the synthesis gas.

6. A reactor according to Claim 5, characterised in that the variation in the spacing c takes place continuously along the flow path of the synthesis gas.

7. A reactor according to Claim 5, characterised in that the spacing c changes sectionwise along the flow path of the synthesis gas and is constant within the sections (17, 18).

Fig.1

Fig.2

Fig.3

Fig.4

Fig.5